# EUROPEAN PATENT APPLICATION

(11) **EP 4 516 144 A1**
(43) Date of publication of application: **05.03.2025**
(21) Application number: 23185684.0
(22) Date of filing: 14.07.2023
(51) Int. Cl.: A41B 9/04, A41D 13/005, A61F 13/496, H05B 3/34

(54) **UNDERWEAR FOR REDUCING MENSTRUAL PAIN AND/OR MENSTRUAL CRAMPS**

(71) Applicant: Génevaux, Franziska, 80337 München (DE); Magerl, Bernhard, 82256 Fürstenfeldbruck (DE); Magerl, Teresa, 82256 Fürstenfeldbruck (DE)
(72) Inventor: Génevaux, Franziska, 80337 München (DE); Magerl, Bernhard, 82256 Fürstenfeldbruck (DE); Magerl, Teresa, 82256 Fürstenfeldbruck (DE)
(74) Representative: Maiwald GmbH

(57) **Abstract**

Underwear (100) for reducing menstrual pain and/or menstrual cramps. The underwear (100) comprises a body (300) including a front part (301), a back part (302), two side parts (303), a pubic part (304) and a waistband (305); and at least one heating element (110) configured to heat the lower part of the torso of a user wearing the underwear (100). The at least one heating element (110) is mushroom-like shaped or T-form-like shaped, wherein the at least one heating element (110) is arranged at least at the front part (301) of the body (300) of the underwear (100).

## Description

### TECHNICAL FIELD

The present disclosure relates to an underwear for reducing menstrual pain and/or menstrual cramps and to an underwear system.

### TECHNICAL BACKGROUND

The general background of this disclosure is the providing of a piece of clothing, in particular underwear, reducing the occurring pain and/or cramps during menstrual periods.

For many menstruating persons, the monthly menstrual period is associated with symptoms such as cramps and pain in the abdomen due to contractions and relaxing of the uterine muscles in an irregular rhythm. The medical term for painful menstrual bleeding is "dysmenorrhea". Medical studies have shown that heat can reduce the cramps and pain in the abdomen.

Hence, there is a need to provide a piece of clothing, in particular underwear, for relieving or reducing the pain and/or cramps during the monthly menstrual period.

### SUMMARY OF THE INVENTION

In one aspect of the invention an underwear for reducing menstrual pain and/or menstrual cramps is presented, comprising:
a body including a front part, a back part, two side parts, a pubic part and a waistband;
at least one heating element configured to heat the lower part of the torso of a user wearing the underwear,
wherein the at least one heating element is mushroom-like shaped or T-form-like shaped, and wherein the at least one heating element is arranged at least at the front part and/or back part of the body of the underwear.

Underwear may be any clothing or any article of clothing worn next to the skin and under other clothing. For instance, the underwear may be a slip, panties, underpants, shorts, but is not limited thereto. Underwear may be provided in different sizes like 34 to 38 and 38 to 44, but is not limited thereto. The underwear comprises a body consisting of a front part, a back part, two side parts, a pubic part, and a waistband. The body of the underwear may comprise or consists of felt, cotton, viscose, linen, polyacryl, polyamide, polyester, silk, wool or mixtures thereof, but is not limited thereto. The waistband is a strip of material that is either elastic or some other confining fabric that encircles the waist of the human body of a user. The front part of the underwear is the part which covers a region of the abdomen extending from the navel to the pubic area. The front part extends from the waistband to the pubic area of a human body, but does not cover the pubic area. The pubic area and the perineum of the human body are covered by the pubic part of the body of the underwear. The pubic part extends from the front part to the back part of the body of the underwear. In other words, the pubic part connects the front part and the back part of the body of the underwear in the pubic area of the human body. The back part of the underwear is the part which covers the bottom and the buttocks of a user. The back part extends from the wasteband to the pubic part. The side parts connect the front part and the back part at the hip of a human body. The user of an underwear may be a person suffering from cramps and pain in the abdomen due to the menstrual period. The lower part of the torso is a body region below the navel at a front side of a body and the lumbar spine at the back side of a human body, wherein the legs are not included in this body region. For instance, the lower part of the torso include the lower abdomen include the pubic area, a right and a left part, in particular quadrant and the lower part of the back, but is not limited thereto. The at least one heating element is to be understood in a broad manner and represents any element which provides heat, i.e. transmits, provides, irradiates infrared (electromagnetically) radiation, to the lower part of the torso of a user. The at least one heating element may comprise at least one layer of carrier material and solely one heating wire or a plurality of heating wires. The at least one heating element may have an electrical power of 2 W to 5 W. In particular, each one or the sum of the at least one heating element may have an electrical power of 2 W to 5 W.The at least one heating element provides heat to the lower part of the torso of a user in a temperature range of 37°C to 50°C, in particular 37°C to 45°C. This temperature range ensures that no burnings on or of the skin of the user occurs. The at least one heating element may have a heating output of 0.01 W/cm² to 0.50 W/cm², preferably 0.15 W/cm², but is not limited thereto. The at least one heating element may have a total area of 100 cm² to 200 cm², preferably 170 cm², but is not limited thereto. In, particular, each one or the sum of the at least one heating element has a total area of 100 cm² to 200 cm², preferably 170 cm², but is not limited thereto.

The at least one heating element comprises a maximum lateral extension, a minimal lateral extension, a maximum vertical extension and a maximum height. The maximum lateral extension, i.e. the width, of the at least one heating element extends parallel to the maximum lateral extension, i.e. width, of the waistband in a non-stretched condition. The maximum lateral extension of the waistband is parallel to the hip size. For instance, the maximum lateral extension of the at least one heating element may be 90 mm to 150 mm, preferably 130 mm, but is not limited thereto. For instance, the minimal lateral extension of the at least one heating element may be 40 mm to 60 mm, preferably 50 mm, but is not limited thereto. In other words, the maximum lateral extension of the at least one heating element may be in a range of 70 % to 90% of the maximum lateral extension, i.e. width, of the waistband. The maximum vertical extension, i.e. the length, of the at least one heating element extends orthogonal to the maximum lateral extension of the waistband, i.e. in the longitudinal axis caudal of the maximum lateral extension of the waistband. For instance, the maximum vertical extension of the at least one heating element may be 150 mm to 190 mm, preferably 170 mm, but is not limited thereto. In other words, the maximum vertical extension of the at least one heating element may be in a range of 70 % to 150%, preferably 110 %, of the maximum lateral extension, i.e. width, of the waistband or may be in a range of 80 % to 150%, preferably 130 %, of the maximum lateral extension, i.e. width, of the at least one heating element. The maximum height of the at least one heating element extends orthogonal to both the maximum lateral extension and the maximum vertical extension. For instance, the maximum height of the at least one heating element may be in a range of 1 mm to 3 mm, but is not limited thereto. The at least one heating element is mushroom-like shaped or T-form-like shaped. In other words, the form of the at least one heating element is based on the biological form of a mushroom or is based on the form of a T. Therefore, the at least one heating element comprises or consists of a cap section, a transition section, and a pillar section in order to provide the mushroom-like shape or T-form-like shape of the at least one heating element. The cap section is directly connected to respectively direct borders at the transition section and the transition section is directly connected to respectively direct borders at the pillar section. The cap section includes the maximum lateral extension of the at least one heating element. The cap section includes the highest proportion of the total area of the at least one heating element, with respect to the proportions of the transition section and/or the pillar section. For instance, the proportion of the cap section of the total area of the at least one heating element may be in a range of 40 % to 60 %. The cap section may have a vertical extension of 30% to 55 % of the maximum vertical extension of the at least one heating element. The form of the cap section may be bulgy, stepped, rectangular, quadratic or trapezoid, in particular isosceles, but is not limited thereto. The form of the cap section may be axially symmetric or axial asymmetric with respect to the longitudinal axle of a human body. The transition section includes the middle or lowest proportion of the total area of the at least one heating element, with respect to the proportions of the cap section and/or the pillar section. For instance, the proportion of the transition section of the total area of the at least one heating element may be in a range of 1 % to 30 %. The transition section may have a vertical extension of 1% to 30 % of the maximum vertical extension of the at least one heating element. The form of the transition section may be stepped, trapezoid, in particular isosceles, but is not limited thereto. The form of the transition section may be axially symmetric or axial asymmetric with respect to the longitudinal axis of a human body. The pillar section includes the minimum lateral extension of the heating element. The pillar section includes the middle or lowest proportion of the total area of the at least one heating element, with respect to the proportions of the cap section and/or the transition section. For instance, the proportion of the pillar section of the total area of the at least one heating element may be in a range of 10 % to 59 %. The pillar section may have a vertical extension of 15 % to 69 % of the maximum vertical extension of the at least one heating element. The form of the pillar section may be bulgy, stepped, rectangular, quadratic or trapezoid, in particular isosceles, but is not limited thereto. The form of the pillar section may be axially symmetric or axial asymmetric, with respect to the longitudinal axle of a human body. The height of the pillar section, transition section and/or cap section can be identical or different to each other. The at least one heating element is arranged at least at the front part and/or back part of the body of the underwear. Alternatively or additionally, the heating element may be arranged at least one of the side parts and/or at the pubic part of the body of the underwear. In a preferred example, solely one heating element is included in the underwear. Alternatively, a plurality, i.e. at least two, heating elements can be comprised in the underwear. The plurality of heating elements can be arranged directly side by side or spaced apart from each other in a sorted/structured or random manner.

By providing heat to the lower part of the torso of a user, in particular a person, the occurring cramps of the uterine muscles during the menstrual period can be relieved, reduced or prevented and therefore the occurring pain can be relieved.

In an embodiment of the underwear, the heating element comprises a cap section, a transition section, and a pillar section, wherein the cap section is arranged adjacent or at the waistband and the transition section and the pillar section extends orthogonally from the cap section.

An arrangement of the cap section adjacent to the waistband represents that the cap section of the at least one heating element is arranged parallel to the waistband with a distance of 0.1 cm to 2 cm at the front part, the back part and/or the side parts of the body of the underwear, but is not limited thereto. An arrangement of the cap section at the waistband represents that the cap section of the at least one heating element is arranged directly at the waistband and at the front part, the back part and/or the side parts of the body of the underwear. In this context, the cap section may be arranged at the waistband completely or at least partially.

By arranging the cap section adjacent or at the waistband, heat can be provided to an area of the lower torso at which most of the users have cramps and/or pain during menstrual periods. Therefore, an optimal respectively ideal providing of heat to the lower torso of a user and an optimal relief or reduction of the cramps and/or pain during menstrual periods can be provided.

In an embodiment of the underwear, an average lateral extension being parallel to the lateral extension of the waistband of the cap section is bigger than an average lateral extension being parallel to the lateral extension of the waistband of the pillar section and/or transition section.

In an embodiment of the underwear, the transition section between the cap section and the pillar section is abrupt for providing the mushroom-like shape or T-form-like shape.

The term abrupt represents that the transition section has the lowest proportion of the total area of the at least one heating element, with respect to the proportions of the cap section and/or the pillar section. Specifically, the proportion of the transition section of the total area of the at least one heating element is in a range of 1 % to 15 %. Further, the transition section may have a vertical extension of 1% to 15 % of the maximum vertical extension of the at least one heating element.

In an embodiment of the underwear, the at least one heating element is releasably or fixedly attached at the front part, the side parts, the back part and/or pubic part of the underwear.

In this context, the heating element can be releasably or fixedly attached at the inner surface, i.e. the side facing the body of a user, or at the outer surface, i.e. the side facing away from the body of a user, of the front part, the side parts, the back part and/or the pubic part of the body of the underwear. Additionally or alternatively, the heating element may be arranged between at least two layers of material at the inner surface forming a tab, i.e. a fully closed or partially closed tab.

The releasable attachment of the heating element to the front part, the side parts, the back part and/or pubic part of the underwear can be provided by a pocket, in which the heating element can be laid in, by a Velcro, in particular hook-and-loop fastener, by at least one zipper, by at least one button and/or by at least one snap fastener, but is not limited thereto. By releasable attaching the heating element to the underwear, the body of the underwear and the heating element can be separated. The separation enables that the body of the underwear and the heating element can be washed separately in different washes, e.g. body of underwear is washed at 60 °C and heating element is washed at 30°C. Further, the possibility of the separation of the body of the underwear and the heating element enables that number of heating elements which has to be bought by the user can be decreased, because e.g. one heating element can be attached to a plurality of bodies of underwear. Therefore, the washability of the underwear, the lifetime of the underwear and the heating element can be increased and the financial impact for a user can be reduced. The fixed attachment of the heating element to the front part, the side parts, the back part and/or pubic part of the underwear can be provided by sewing or stitching the heating element to the front part, the back part, the side parts and/or pubic part. Alternatively or additionally, the heating element can be glued or stacked to the heating element to the front part, the side parts, the back part and/or pubic part of the body of the underwear. By fixedly attaching the heating element to the underwear, a handling of the underwear during storing the underwear in a closet or during washing of the underwear can be simplified. Further, a fixed attachment of the heating element prevents a shifting of the heating element whilst putting on the underwear by a user. Therefore, the handling of the underwear can be simplified and the optimal arrangement of the heating element for providing heat to the lower part of the torso/pelvic region of a user can be ensured.

In an embodiment of the underwear, the heating element comprises a carrier material and at least one heating wire extending in a two-dimensional winding form across the carrier material, wherein the extending of the at least one heating wire in the two-dimensional winding form across the carrier material provides the mushroom-like shape or T-form-like shape of the at least one heating element.

The carrier material may be any material on which or in which the at least one heating wire is arranged or inserted respectively inlaid. For instance, the carrier material may be felt, cotton, viscose, linen, polyacryl, polyamide, polyester, silk, wool or mixtures thereof, but is not limited thereto. Additionally, the carrier material may include heat dissipating fibers. The heat dissipating fibers may be arranged in a two-dimensional grid structure, but is not limited thereto. The heat dissipating fibers may comprise or consist of a material having a heat coefficient > 1 W/(mK). An arrangement of the at least one heating wire on the carrier material may be provided in a fixed manner by sticking, in particular gluing, or sewing, in particular stitching. An arrangement of the at least one heating wire in the carrier material may be provided by sandwiching the at least one heating wire between two identical or different layers of carrier material. The heating wire may be any element, in particular wire or cable, which converts electrical energy into heat through the process of Joule heating, but is not limited thereto. Electric current through the element encounters resistance, resulting in heating of the element. This process is independent of the direction of current. For instance, the heating wire may comprise a core including a nichrome 80/20 (80% Nickel, 20% Chromium) wire, ribbon, or strip, a Cantal (FeCrAl) wire or a Cupronickel (CuNi) alloy, but is not limited thereto. The core may have a diameter of 0.5 mm to 3 mm, but is not limited thereto. Further, the heating wire may comprise an isolation in which the metal core is inserted. The insulation may be made of plastic and may have a thickness of 0.5 mm to 3 mm, but is not limited thereto. The heating wire may be configured for a voltage of 2 V to 10 V, preferable 5 V, but is not limited thereto. The two-dimensional winding form as used herein is to be understood broadly and represents any two-dimensional form of the at least one heating wire being arranged with windings. For instance, the two-dimensional winding form may be a two-dimensional meander form, a two-dimensional double meander form or a two-dimensional bifilar spiral form, but is not limited thereto. Additionally, the two-dimensional winding form may include one or a plurality of waves and/or the windings are arranged in an axially symmetric or axial asymmetric manner, i.e. windings extend in such a manner that their extent is axial symmetric with respect to a virtual axis extending between the windings. The two-dimensional winding form in the at least one heating element may include 2 to 15, preferably 9, windings, in particular meander loops or number of spirals, across the carrier material and therefore the heating element. The windings may be arranged with a constant, a non-constant or a hybrid constant distance, i.e. partially constant distance and partially non-constant distance, with respect to each other. The at least one heating wire of the two-dimensional winding form may cover 25 % to 60 %, preferably 35%, of the area of the carrier material and therefore the area of the at least one heating element.

By arranging the heating wire in a two-dimensional winding form, the area of the heating element covered by the at least one heating wire can be maximized such that an optimal respectively ideal providing of heat to the lower part of the torso of a user and an optimal relief or reduction of the cramps and/or pain during menstrual periods can be provided.

In an embodiment of the underwear, the at least one heating wire comprises a carrier material, a first heating wire and at least one second heating wire, wherein the first heating wire and the at least one second heating wire are different to each other.

In this context, the first heating wire and the at least one second heating wire may differ from each other in e.g. the used material of the core, the diameter of the core, the thickness of the insulation and/or the configuration of the wire regarding used voltage, but is not limited thereto.

By providing a plurality of different heating wires in the at least one heating element, a plurality of heating zones can be provided.

In an embodiment of the underwear, the extending of the at least one heating wire in the two-dimensional winding form across the carrier material comprises that the heating wire meanders or extends wave-like in a plurality of rows in transverse direction of the waistband of the underwear.

In this context, transverse direction represents that the heating wire meanders or extends wave-like in a plurality of rows in a first direction being parallel to the lateral extension of the waistband and/or in a second direction being orthogonal to the lateral extension, but is not limited thereto.

By arranging the heating wire in a two-dimensional winding form, the area of the heating element covered by the at least one heating wire can be maximized such that an optimal respectively ideal providing of heat to the lower torso of a user and an optimal relief or reduction of the cramps and/or pain during menstrual periods can be provided.

In an embodiment of the underwear, the extending of the at least one heating wire in the two-dimensional winding form across the carrier material in the cap section is different to the extending of the at least one heating wire in the two-dimensional winding form across the carrier material in the pillar section and/or transition section.

In this context, the extending of the at least one heating wire in the two-dimensional winding form in the cap section, in the pillar section and/or in the transition section may differ in the type of two-dimensional winding form, i.e. e.g. two-dimensional meander form, two-dimensional double meander form or two-dimensional bifilar spiral form, in the including of the number of waves, in the arrangement of the windings in an axially symmetric or axial asymmetric manner, in the number of windings across the carrier material and therefore the heating element, in the distance of the windings with respect to each other and/or in the covered area of the carrier material by the at least one heating wire, but is not limited thereto.

By using different extending of the at least one heating wire in the two-dimensional winding form in the cap section, in the pillar section and/or in the transition section, a plurality of different heating zones can be provided. Therefore, an optimal respectively ideal providing of heat to the lower torso of a user and an optimal relief or reduction of the cramps and/or pain during menstrual periods can be provided.

In an embodiment of the underwear, the at least one heating wire in the cap section is different to the at least one heating wire in the pillar section and/or transition section.

In this context, the at least one heating wire in the cap section may differ from the at least one heating wire in the pillar section and/or transition section in e.g. the used material of the core, the diameter of the core, the thickness of the insulation and/or the configuration of the wire regarding used voltage, but is not limited thereto.

By using different heating wires in the cap section, in the pillar section and/or in the transition section, a plurality of different heating zones can be provided. Therefore, an optimal respectively ideal providing of heat to the lower torso of a user and an optimal relief or reduction of the cramps and/or pain during menstrual periods can be provided.

In an embodiment of the underwear, the heating element has an electrical power of 2 W to 5 W.

In an embodiment of the underwear, the heating element provides heat to the lower part of the torso of a user in a temperature range of 37°C to 50°C, in particular 37°C to 45°C.

By providing heat in a temperature range of 37°C to 50°C, in particular 37°C to 45°C, an optimal respectively most efficient heat can be provided to the lower part of the torso of a user, such that the occurring cramps of the uterine muscles during the monthly menstrual period can be relieved, reduced or prevented and therefore the occurring pain can be relieved.

In an embodiment of the underwear, the underwear further comprises at least one connector for releasable attaching and electrical connecting a power supply unit for providing power to the heating element.

The connector may be any device providing an electrical connection for providing power to the heating element and/or a physical connection between the at least one power supply unit and to the underwear. For instance, the connector may be one part of a magnet fastener, a hook-and-eye closure, a snap fastener and/or a Velcro, but is not limited thereto. The connector comprises at least one material having an electrically conductivity of >10⁶ S/m. In order to provide an electrical connection for providing power to the heating element and/or a physical connection of one or a plurality of different or same connector types as mentioned above can be used. Alternatively, the connector may also be a plug-socket system, like a Universal Serial Bus, USB, system. The power supply unit may be any device which is capable to provide power to the heating element. For instance, the power supply unit may be a power storage unit, a battery, or an accumulator. The accumulator may be a 1A ,5V accumulator, preferably 3.7V accumulator. Additionally, the accumulator may be a 4000 mAh which has accumulator operation time of 4 hours and a weight of 70 grams. Alternatively, the accumulator may be an 8000 mAh which has accumulator operation time of 8 hours and a weight of 135 grams. The power supply unit may comprise a casing being hermetically isolated and heat resistant up to 120°C, such that the power supply unit can be washed together with the underwear.

By providing a connector which provides a physical and/or electrical connection, the complexity of the connection can be decreased and the handling of the underwear can be simplified.

In an embodiment of the underwear, the connector is arranged at the back part of the underwear.

In a preferred embodiment of the underwear, the connector is arranged at a transition of the back part to one side part of the body of the underwear. Alternatively, the connector can be freely arranged, i.e. not connected to the body of the underwear, or arranged at a location being different to the back part.

By arranging the connector at the back part of the underwear, the wearing comfort of the underwear can be increased, because the connector cannot be felt or perceived while wearing the underwear, i.e. laying, walking, and sitting with the underwear.

In an embodiment of the underwear, the at least one connector and the at least one heating element are electrically connected by at least one wire.

In this context, the wire may be any elongated electrically conductive material which provides an electrical connection between the at least one connector and the at least one heating element. For instance, the wire may be a cable or an electronically conductive band respectively foil having a straight or a spiral form. The wire may have a length of 25 cm to 50 cm, but is not limited thereto. In case the wire is a cable, the cable may be a two-core cable or a multi-core, i.e. more than 2, cable, wherein each one of the cores may be a cable in the range of 0.82 mm² to 0,05 mm², but not limited thereto. The wire can be arranged in the waistband, in a separate "cable channel" in the body of the underwear, and/or freely within the underwear.

By connecting the at least one connector with the at least one heating element by at least one wire, an electrical connection between the at least one connector and the at least one heating element is reliably provided.

In an embodiment of the underwear, the at least one connector is a magnet fastener, a hook and eye closure, a snap fastener and/or a Velcro.

By using a magnet fastener, a hook-and-eye closure, a snap fastener and/or a Velcro, in particular a hook-and-loop fastener, as the at least one connector, an easy and reliable attachment, fixation or mounting of components being attached to the connector can be provided. Additionally, by using of a magnet fastener, a hook-and-eye closure, a snap fastener and/or a Velcro, in particular a hook-and-loop fastener, as the at least one connector, an electrical connectivity between the connector and the components to be attached to the connector can be provided in an easy and reliable manner. Therefore, the ease of use can be increased.

In an embodiment of the underwear, the underwear further comprises a control unit for controlling the at least one heating element.

The control unit may be any unit with which the at least one heating element can be controlled. The controlling of the at least one heating element comprises the modes/programs for turning on or off of the heating element, for controlling or regulating of the provided temperature to the lower part of the torso of the user, for controlling of the power consumption of the heating element, for setting a timer for providing at least one heating interval, and/or one or for selecting one of a plurality of different pre-set heating intervals, but is not limited thereto. The at least one heating interval or the plurality of heating intervals may be stored in a memory being included in the control unit. When the at least one heating element comprises a plurality of heating zones, the control unit is also capable to control the above mentioned heating modes/programs for all heating zones separately. Further, the control unit may comprise solely one or a plurality of push buttons, with which at least one of the above mentioned heating modes/programs can be selected and set. Alternatively or additionally, the control unit may be controlled by a remote control or a mobile phone. In this context, the control unit may include a Bluetooth and/or Wi-Fi connection to a mobile device. The control unit may be included in or at the body of the underwear or at the power supply unit. In case the control unit is included in or at the body of the underwear, the control unit is connected/arranged between the connector and the heating element. In this context, the control unit may be hermetically isolated and heat resistant up to 120°C, such that the body of the underwear can be washed together with the control unit. In case the control unit is included at the power providing unit, the control unit is connected/arranged between the power providing unit and the complementary connector of the connector being included in or at the underwear. In this context, the control unit can be also hermetically isolated. Additionally, the control unit may comprise at least one sensor measuring the temperature of the skin and/or the body of the user for preventing an overheating of the user of the underwear. The preventing of an overheating of the user can be provided because the controlling of the heating element is controlled by the measured temperature of the skin and/or the body of the user. For instance, when the sensor detects that the temperature of the skin and/or the body of the user is above a pre-defined threshold, the control unit will automatically reduce the provided temperature in order to prevent overheating. The at least one sensor may be arranged in direct or indirect contact to the skin of the user.

By including a control unit to the underwear, the provided quantity of the heat to the lower part of the torso can be selected and controlled such that an optimal respectively ideal providing of heat to the lower part of the torso of a user and an optimal relief or reduction of the cramps and/or pain during menstrual periods can be provided.

In a further aspect, an underwear system is presented. The underwear system comprises an underwear as described above and a power supply unit for providing power to the at least one heating element.

By providing a system comprising of an underwear which provides heat to the lower part of the torso of a user and a power supply unit, the occurring cramps of the muscles of the uterus during the monthly menstrual period can be relieved, reduced or prevented and therefore the occurring pain can be relieved.

In a further aspect, an underwear system is presented. The underwear system comprises an underwear as described above and a power supply unit for providing power to the at least one heating element, wherein the power supply unit comprises a control unit for controlling the at least one heating element.

By providing a system comprising of an underwear which provides heat to the lower part of the torso of a user and a power supply unit, the occurring cramps of the muscles of the uterus during the monthly menstrual period can be relieved, reduced or prevented and therefore the occurring pain can be relieved.

In an embodiment of the underwear system, the power supply unit is arranged and electrically connected directly at the connector or is arranged separate to the connector and electrically connected by wire to the connector.

By arranging and electrically connecting the power supply unit directly at the connector, the wearing comfort of the underwear can be increased, because the connector and the power supply unit cannot be felt or perceived during wearing the underwear, i.e. walking and sitting with the underwear. Further, the power drop due to the non-used wire can be prevented. By arranging the power supply unit separate to the connector and electrically connecting the power supply unit by wire to the connector, the wearing comfort of the underwear can be increased, because the connector and the power supply unit cannot be felt or perceived during wearing the underwear, i.e. walking and sitting with the underwear.

Any disclosure and embodiments described herein relate to the underwear and the underwear system, lined out above and vice versa. Advantageously, the benefits provided by any of the embodiments and examples equally apply to all other embodiments and examples and vice versa.

### BRIEF DESCRIPTION OF THE DRAWINGS

In the following, the present disclosure is further described with reference to the enclosed figures:
- Fig. 1: illustrates an example embodiment of the underwear;
- Fig. 2: illustrates a detail view of an example embodiment of the heating element;
- Fig. 3: illustrates a top view of the body of the underwear,
- Fig. 4: illustrates another example embodiment of the underwear;
- Fig. 5: illustrates an external view on the underwear of an example embodiment of the connector;
- Fig. 6: illustrates an internal view on the underwear of an example embodiment of the connector;
- Fig. 7: illustrates an example embodiment of the power supply unit;
- Fig. 8: illustrates an example embodiment of the underwear system.

### DETAILED DESCRIPTION OF EMBODIMENT

The following embodiments are mere examples for the underwear and the underwear system disclosed herein and shall not be considered limiting.

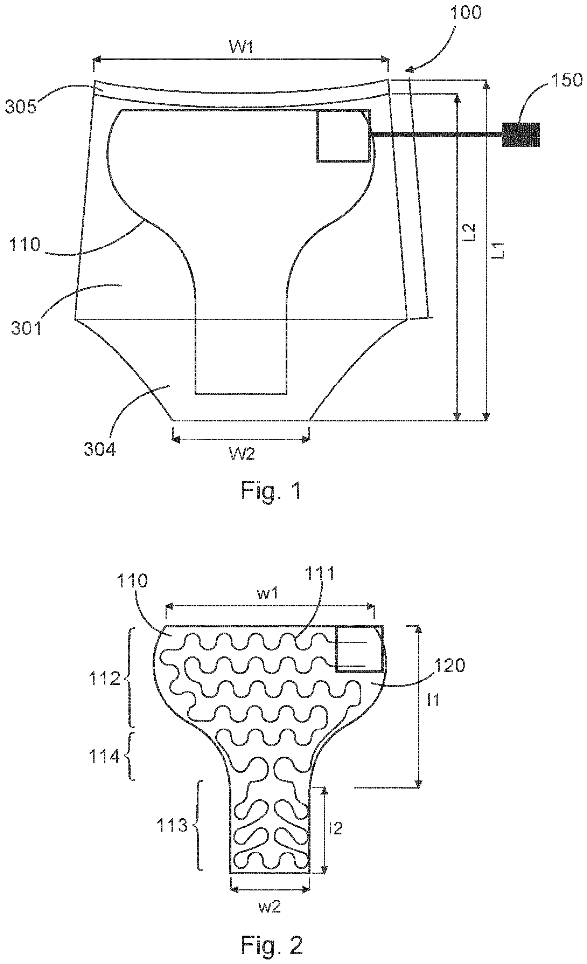

Fig. 1 illustrates an example embodiment of the underwear. The underwear 100 comprises at least one, in particular one, heating element 110. The heating element 110 is arranged at the front part 301 and at the pubic part 304 of a body 300 of an underwear 100. Further, the heating element 110 is arranged directly and at least partially at the waistband 305 of the body 300 of the underwear 100. The heating element 110 is connected via a wire to a connector 150, wherein the connector 150 provides power to the heating element 110. In this context, the connector 150 is a snap fastener or USB which is freely arranged, i.e. not arranged at the body 300 of the underwear 100. The width of the waistband 305 represents an upper width W1 of the body 300 of the underwear 100. The pubic area 304 comprises a lower width W2 of the body 300 of the underwear 100. L1 is the total length of the body 300 of the underwear 100 and L2 is the length of the body 300 of the underwear 100 without the width of the waistband 305. In other words L2 is the maximum length that the heating element 110 can have.

Fig. 2 illustrates a detail view of an example embodiment of the heating element 110. The at least one heating element 110 may have a total area of 170cm². The maximum lateral extension w1 of the at least one heating element 110 is 130 mm. The minimal lateral extension w2 of the at least one heating element 110 is 50 mm. The maximum vertical extension, i.e. l1 + l2, of the at least one heating element 110 is 170 mm. The maximum height or thickness of the at least one heating element 110 is 2 mm. The heating element 110 comprises one heating wire 111 being a closed loop. The heating wire 111 extends respectively meanders laterally, i.e. parallel to the waistband, in the transverse direction. The heating wire 111 has a two-dimensional winding form, in particular a meander form, having additional waves. The additional waves may be sinus or cosinus waves. The heating wire 111 comprises six meander windings which extends across the carrier material 120 and provides the mushroom-like shape of the heating element 110. Further, the heating wire 111 comprises thirty eight additional waves. Two of the six meander windings are arranged in the cap section 112 of the heating element 110. One of the six meander windings are arranged in the transition section 114 of the heating element 110. Three of the six meander windings are arranged in the pillar section 113 of the heating element 110. Twenty one of the thirty eight additional windings are arranged in the cap section 112. Nine of the thirty eight additional winding are arranged in the transition section 114. Eight of the thirty eight additional winding are arranged in the pillar section 113. The proportion of the cap section 112 of the total area of the at least one heating element 110 is 55%. The cap section 112 has a vertical extension of 55 % of the maximum vertical extension of the at least one heating element 110. The form of the cap section 112 is bulgy. The form of the cap section 112 is axially symmetric with respect to the longitudinal axis of a human body. The transition section 114 has the lowest proportion of the total area of the at least one heating element 110, with respect to the proportions of the cap section 112 and/or the pillar section 113. The proportion of the transition section 114 of the total area of the at least one heating element 110 is 15 %. The transition section 114 has a vertical extension 15 % of the maximum vertical extension of the at least one heating element 110. The form of the transition section 114 is trapezoid and is axially symmetric with respect to the longitudinal axis of a human body. The pillar section 113 has the middle proportion of the total area of the at least one heating element 110, with respect to the proportions of the cap section 112 and/or the transition section 114. The proportion of the pillar section 113 of the total area of the at least one heating element 110 is 30 %. The pillar section 113 has a vertical extension of 30 % of the maximum vertical extension of the at least one heating element 110. The form of the pillar section 113 is rectangular. The form of the pillar section 113 is axially symmetric with respect to the longitudinal axis of a human body. The height or thickness of the pillar section 113, transition section 114 and cap section 112 are identical. The width w1 and w2 of the heating element 110 is smaller than the width W1 and W2 of the body 300 of the underwear 100, in particular the respective parts of the underwear 100. The heating wire 111 comprises a core including a nichrome 80/20 (80% Nickel, 20% Chromium) wire. The core has a diameter of 1 mm. Further, the heating wire comprises an isolation in which the metal core is inserted. The insulation may be made of plastic and has a thickness of 0.5 mm. The heating wire is configured for a voltage of 5 V. The two-dimensional winding form of the cap section 112, the two-dimensional winding form of the transition section 114, and the two-dimensional winding form of the pillar section 113 are different to each other. The heating wire 111 is glued or stiched on a carrier material 120. The carrier material 120 is made of felt.

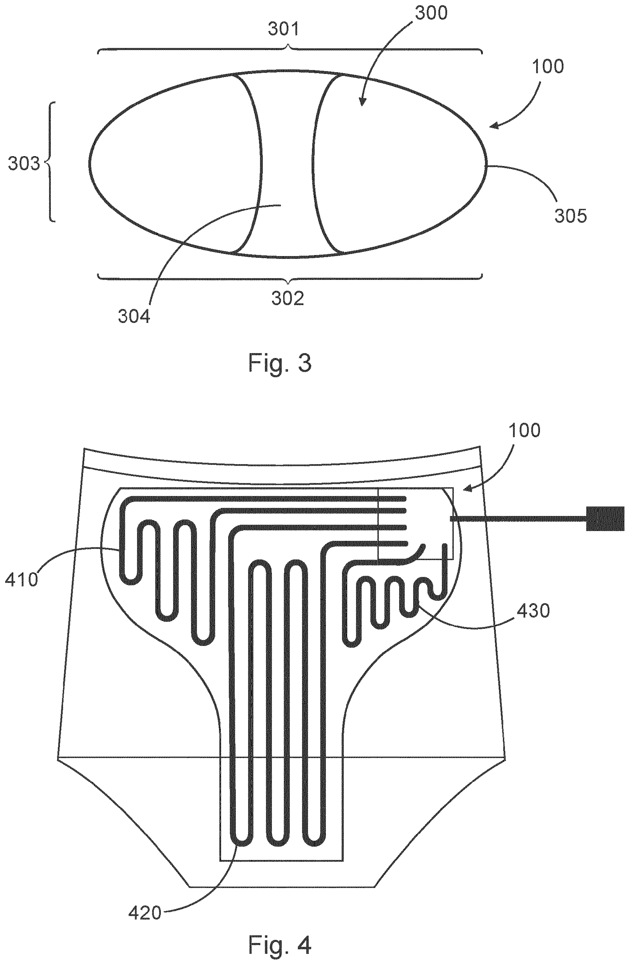

Fig. 3 illustrates a top view of the body 300 of the underwear 100. The underwear 100 comprises a body 300. The body 300 comprises a front part 301 and a back part 302. The front part 301 and the back part 302 are physically connected to each other by two side parts 303, each being arranged at the hip bone level, and by a pubic part 304 being arranged respectively covering the pubic area of a human body. A waistband 305 encircles the waist of the human body of a user and is connected to the front part 301, to the back part 302, and to both of the two side parts 303. The body of the underwear, i.e. the front part 301, the back part 302, the side parts 303, and the pubic part 304 are made of cotton. The waistband is made of an elastic respectively stretchable material band.

Fig. 4 illustrates another example embodiment of the underwear. In particular, Fig. 4 depicts another example of the heating element as depicted in Fig. 2. In contrast to the example embodiment of the heating element 110 as depicted in Figure 2, the heating element 110 as depicted in Figure 4, comprises a first heating wire 420, a first second heating wire 410, and a second second heating wire 430. The heating wires are extended in a meander form across the carrier material 120 in such a manner that the heating wires 420, 410 and 430 extend in a direction being orthogonal to the lateral extension of the waistband 305. The three heating wires 420, 410 and 430 are identical, i.e. these wires have the same diameter etc.

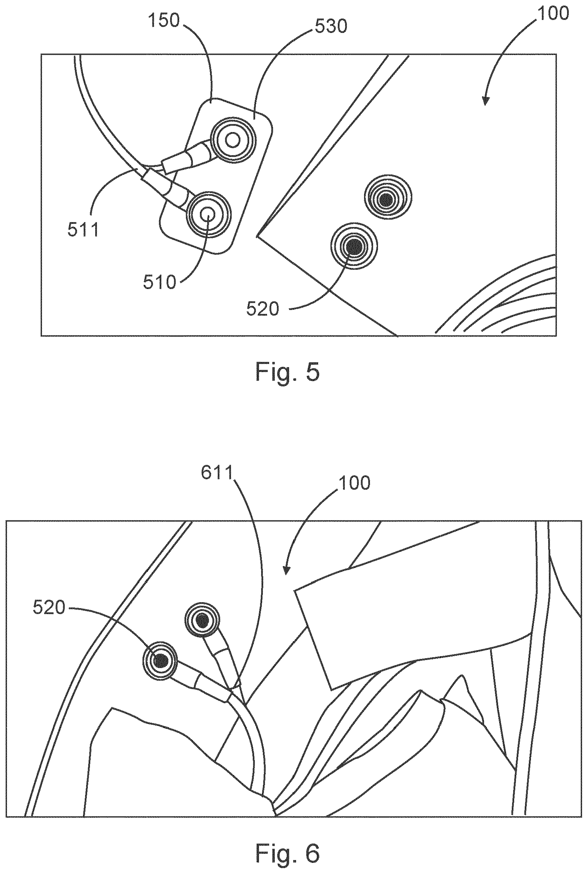

Fig. 5 illustrates an external view on the underwear 100 of an example embodiment of the connector 150. The connector 150 is a snap fastener. The snap fastener comprises a "female" part 510 and a "male" part 520. The "female" part 510 comprises a socket and a cap being arranged at a strap 530 being made of leather. At the strap 530 two "female" parts 510 are arranged, wherein both "female" parts 510 are electrically connected to a wire 511. The wire 511 provides electrical power from a power supply unit to the "female" parts 510 of the connector. One of these "female" parts 510 is electrically connected to a positive pole of the power supply unit and the other "female" part 510 is electrically connected to a negative pole of the power supply unit. The "female" part 510 and a "male" part 520 of the connector 150 is made of aluminum or brass having an electrically conductivity of 3,7 * 10⁷ S/m. At or on the underwear 100, in particular at the body of the underwear 100, also two "male" parts 520 of the connector 150 are arranged. Specifically, the "male" parts 520 of the connector 150 are arranged at the back part 302 at the transition of the back part 302 to one side part 303. The "male" parts 520 comprise an eyelet and a stud. Both of the "female" parts 510 and both of the "male" parts 520 are arranged in a complementary manner, such that both parts can be easily connected without inducing any mechanical stress.

Fig. 6 illustrates an internal view on the underwear 100 of an example embodiment of the connector 150. Both "male" parts 520 of the connector 150 are electrically connected to a wire 611. The wire 611 provides electrical power from the connector 150 to the heating element 110. The wire 611 is arranged freely in the underwear, i.e. neither in the waistband nor in a separate "cable channel" in the body of the underwear 100. The "male" parts 520, in particular the eyelet of the "male" parts which are in direct contact with the skin of a user are electrically isolated, in order to prevent short circuits on the skin of the user.

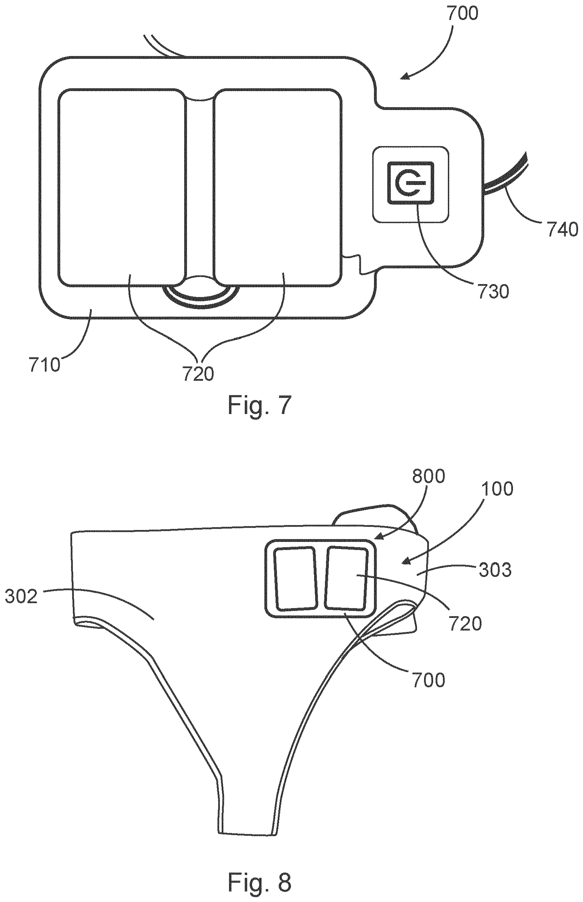

Fig. 7 illustrates an example embodiment of the power supply unit 700. The power supply unit 700 comprises two accumulators 720. Each one of the accumulators 720 is a 2000 mAh accumulator, which has an accumulator operation time of 4 hours and a weight of 70 grams each. The power supply unit 700 also comprises a control unit 730. The control unit 730 is a button with which a current flow from the power supply unit 700 to a connector through the wire 740 can be provided. The power supply unit 700, i.e. both the accumulators 720 and also the control unit 730, is included in a casing 710. The casing 710 hermetically isolates the components of the power supply unit 700, such that the power supply unit 700 can be washed together with the underwear 100. The casing 710 is heat resistant up to 120°C.

Fig. 8 illustrates an example embodiment of the underwear system. The underwear system 800 comprises the underwear 100 and the power supply unit 700. The power supply unit 700 comprising two accumulators 720, is arranged at the back part 302 at the transition of the back part 302 to one side part 303, in particular the right side part, of the body 300 of the underwear 100. The power supply unit 700 is electrically and physically connected to the underwear by a connector being a snap fastener.

The present disclosure has been described in conjunction with a preferred embodiment as examples as well. However, other variations can be understood and effected by those persons skilled in the art and practicing the claimed invention, from the studies of the drawings, this disclosure and the claims.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

### Reference signs list:

100 underwear
110 heating element
111 heating wire
112 cap section
113 pillar section
114 transition section
120 carrier material
150 connector
300 body
301 front part
302 back part
303 side part
304 pubic part
305 waistband
410 first second heating wire
420 first heating wire
430 second second heating wire
510 "female" part of the connector
511 wire
520 "male" part of the connector
611 wire
700 power supply unit
710 casing
720 accumulator
730 control unit
740 wire
800 underwear system
W1 upper width of the body of the underwear
W2 lower width of the body of the underwear
L1 length of the body of the underwear
L2 length of the body of the underwear without waisteband
w1 upper width of the heating element
w2 lower width of the heating element
l1 length of the cap section and transition section
l2 length of the pillar section

## Claims

1. Underwear (100) for reducing menstrual pain and/or menstrual cramps, comprising:
a body (300) including a front part (301), a back part (302), two side parts (303), a pubic part (304) and a waistband (305);
at least one heating element (110) configured to heat the lower part of the torso of a user wearing the underwear (100),
wherein the at least one heating element (110) is mushroom-like shaped or T-form-like shaped,
wherein the at least one heating element (110) is arranged at least at the front part (301) and/or the back part (302) of the body (300) of the underwear (100).

2. Underwear (100) according to claim 1,
wherein the at least one heating element (110) comprises a cap section (112), a transition section (114), and a pillar section (113),
wherein the cap section (112) is arranged adjacent or at the waistband (305) and the transition section (114) and the pillar section (113) extends orthogonally from the cap section (112).

3. Underwear (100) according to claim 2,
wherein an average lateral extension being parallel to the lateral extension of the waistband (305) of the cap section (112) is bigger than an average lateral extension being parallel to the lateral extension of the waistband (305) of the pillar section (113) and/or transition section (114).

4. Underwear (100) according to any one of claims 2 or 3,
wherein the transition section (114) between the cap section (112) and the pillar section (113) is abrupt for providing the mushroom-like shape or T-form-like shape.

5. Underwear (100) according to any one of the preceding claims,
wherein the at least one heating element (110) is releasably or fixedly attached at the front part (301), the side parts (303), the back part (302) and/or the pubic part (304) of the underwear (100).

6. Underwear (100) according to any one of the preceding claims,
wherein the at least one heating element (110) comprises a carrier material (120) and at least one heating wire (111) extending in a two-dimensional winding form across the carrier material (120), wherein the extending of the at least one heating wire (111) in the two-dimensional winding form across the carrier material (120) provides the mushroom-like shape or T-form-like shape of the at least one heating element (110).

7. Underwear (100) according to claim 6,
wherein the at least one heating element (110) comprises a carrier material (120), a first heating wire (420) and at least one second heating wire (410),
wherein the first heating wire (420) and the at least one second heating wire (410) are different to each other.

8. Underwear (100) according to any one of claims 6 or 7,
wherein the extending of the at least one heating wire (111) in the two-dimensional winding form across the carrier material (120) comprises that the at least one heating wire (111) meanders or extends wave-like in a plurality of rows in transverse direction of the waistband (305) of the underwear (100).

9. Underwear (100) according to any one of claims 6 to 8,
wherein the extending of the at least one heating wire (111) in the two-dimensional winding form across the carrier material (120) in the cap section (112) is different to the extending of the at least one heating wire (111) in the two-dimensional winding form across the carrier material (120) in the pillar section (113) and/or transition section (114).

10. Underwear (100) according to any one of claims 6 to 9,
wherein the at least one heating wire (111) in the cap section (112) is different to the at least one heating wire (111) in the pillar section (113) and/or transition section (114).

11. Underwear (100) according to any one of the preceding claims,
wherein the at least one heating element (110) has an electrical power of 2 W to 5 W.

12. Underwear (100) according to any one of the preceding claims,
wherein the at least one heating element (110) provides heat to the lower part of the torso of a user in a temperature range of 37°C to 50°C.

13. Underwear (100) according to any one of the preceding claims, further comprising:
at least one connector (150) for releasable attaching and electrically connecting a power supply unit (700) for providing power to the at least one heating element (110).

14. Underwear (100) according to claim 13,
wherein the at least one connector (150) is arranged at the back part (302) of the underwear (100).

15. Underwear (100) according to any one of claims 13 or 14,
wherein the at least one connector (150) and the at least one heating element (110) are electrically connected by at least one wire (111).

16. Underwear (100) according to any one of claims 13 to 15,
wherein the at least one connector (150) is a magnet fastener, a hook and eye closure, a snap fastener and/or a Velcro.

17. Underwear (100) according to any one of the preceding claims, further comprising:
a control unit (730) for controlling the at least one heating element (110).

18. Underwear system (800) comprising:
an underwear (100) according to claims 1 to 17,
a power supply unit (700) for providing power to the at least one heating element (110).

19. Underwear system (800) comprising:
an underwear (100) according to claims 1 to 16,
a power supply unit (700) for providing power to the at least one heating element (110),
wherein the power supply unit (700) comprises a control unit (730) for controlling the at least one heating element (110).

20. Underwear system (800) according to claim 18 or 19,
wherein the power supply unit (700) is arranged and electrically connected directly at the connector (150) or is arranged separate to the connector (150) and electrically connected by wire (740) to the connector (150).
